# EUROPÄISCHE PATENTSCHRIFT

(11) **EP 3 354 351 B1**
(45) Veröffentlichungstag und Bekanntmachung des Hinweises auf die Patenterteilung: **14.08.2019**
(21) Anmeldenummer: 18163697.8
(22) Anmeldetag: 18.09.2013
(51) Int. Cl.: B05B 11/00, A61M 15/08

(54) **SPENDER MIT KINDERSICHERUNG**
DISPENSER WITH CHILD-PROOF PROTECTION SYSTEM
DISTRIBUTEUR À SÉCURITÉ ENFANT

(30) Priorität: 10.10.2012 DE 102012218434
(43) Veröffentlichungstag der Anmeldung: 01.08.2018
(62) Teilanmeldung aus: 13184893.9
(73) Patentinhaber: Aptar Radolfzell GmbH, 78315 Radolfzell (DE)
(72) Erfinder: Greiner-Perth, Jürgen, 78244 Gottmadingen (DE); Szymiczek, Christoph, 78224 Singen (DE)
(74) Vertreter: Patentanwaltskanzlei Cartagena

(56) Entgegenhaltungen:
- DE-A1-102010 011 761
- US-A1- 2003 106 901

## Beschreibung

Die Erfindung betrifft einen Spender zum Austrag eines flüssigen oder pulverförmigen Mediums mit einem Mediumspeicher, mit einer Austragöffnung und mit einer Fördereinrichtung zur Förderung von Medium vom Mediumspeicher zur Austragöffnung. Dabei weist ein gattungsgemäßer Spender eine Betätigungshandhabe der Fördereinrichtung auf, die gegenüber dem Mediumspeicher in einer Betätigungsrichtung verlagerbar ist, um hierdurch einen Austrag des Mediums zu bewirken.

Derartige Spender sind aus dem Stand der Technik allgemein bekannt. Rein beispielhaft wird auf die DE 10 2004 050 679 A1 verwiesen, aus der ein Nasalspender bekannt ist.

Derartige Spender dienen dem Austrag pharmazeutischer Medien. Es ist daher häufig gewünscht, die Zugänglichkeit des Mediums für Kinder zu erschweren, so dass diese nicht versehentlich beim Spielen mit möglicherweise schädlichen Medien in Kontakt kommen.

Aus dem Stand der Technik sind verschiedene Möglichkeiten für kindergesicherte Spender bekannt. So schlägt die DE 10 2009 049 903 A1 beispielsweise zum Einen Spender vor, die bei aufgesetzter Kappe nicht betätigt werden können, da ihre Betätigungshandhabe durch die Kappe blockiert wird. Zum Anderen nennt die genannte Schrift Kappengestaltungen, die nur durch ungewöhnliche Bewegungsabläufe vom jeweiligen Spender zu trennen sind.

Viele der bekannten Gestaltungen von kindergesicherten Spendern betreffen Spender, die bereits originär als kindergesicherte Spender konzipiert worden sind. Derartige kindergesicherte Spender sind jedoch in der Herstellung vergleichsweise teuer, da ihre Teilkomponenten meist nicht für andere möglicherweise nicht kindergesicherte Spender verwendbar sind, so dass eigene Werkzeuge für mit meisten Teilkomponenten solcher kindergesicherter Spender erforderlich sind.

Aus der US 2003/0106901 A1 ist ein Spender bekannt, der über eine Austragöffnung verfügt, die mittels einer schwenkbaren Klappe verschließbar ist. Diese Klappe verhindert dabei über einen Fortsatz, der im geschlossenen Zustand der Klappe in eine Vertiefung eines Sprühkopfes eintaucht, das Niederdrücken des Sprühkopfes.

Aus der DE 102010011761 A1 ist eine Abgabevorrichtung bekannt, die über eine interne Verriegelungsklinke verfügt, mittels derer ein Niederdrücken einer Betätigungshandhabe verhindert werden kann.

### AUFGABE UND LÖSUNG

Aufgabe der Erfindung ist es, hierzu eine Alternative zur Verfügung zu stellen, nämlich Gestaltungen kindergesicherter Spender, die in nur geringem Maße den Grundaufbau des Spenders betreffen, so dass der Spender bei nur geringfügigen Anpassungen sowohl als kindergesicherter als auch als nicht kindergesicherter Spender hergestellt werden kann.

Diese Aufgabe wird erfindungsgemäß wie folgt gelöst. Erfindungsgemäß ist bei einem Spender vorgesehen, dass dieser eine Sicherungsvorrichtung aufweist, die über ein Hauptsegment und ein Schaltsegment verfügt. Das Hauptsegment seinerseits besteht aus einem Basisabschnitt und mindestens einem gegenüber dem Basisabschnitt schwenkbaren Sicherungsausleger. Das Schaltsegment ist dabei gegenüber dem Hauptsegment zwischen einer Sicherungsposition und einer Freigabeposition verlagerbar. Dabei limitiert das Schaltsegment die Beweglichkeit des Sicherungsauslegers des Hauptsegments in der Sicherungsposition, während in der Freigabeposition keine funktionale Limitierung gegeben ist. Diese Sicherungsvorrichtung als Ganzes ist derart an der Fördereinrichtung anbringbar oder fest an dieser angebracht, dass bei Sicherung des Hauptsegments mittels des Schaltsegments die Zugänglichkeit und/oder die Beweglichkeit der Betätigungshandhabe gegenüber dem Mediumspeicher verhindert wird und/oder die Sicherungsvorrichtung nicht von der Fördereinrichtung trennbar ist. Dagegen ist durch Verlagerung des Schaltsegments in die Freigabeposition und die dadurch erzielbare Beweglichkeit des Sicherungsauslegers gegenüber dem Basissegment die Zugänglichkeit oder Beweglichkeit der Betätigungshandhabe herstellbar und/oder je nach Ausgestaltung die Sicherungsvorrichtung als Ganzes von der Fördereinrichtung trennbar.

Die gemäß dieser zweiten Variante vorgesehene Sicherungsvorrichtung weist somit zwei Hauptbestandteile auf, nämlich das Hauptsegment und das Schaltsegment. Der Basisabschnitt der Hauptsegments kann ortsfest zur Betätigungshandhabe oder dem Mediumspeicher am Spender angebracht werden. Der demgegenüber verschwenkbare Sicherungsausleger kann dann in einer Schwenkposition in der beschriebenen Art und Weise die Beweglichkeit oder Zugänglichkeit der Betätigungshandhabe durch Zusammenwirken mit der Betätigungshandhabe oder anderen Komponenten des Spenders verhindern. In einer demgegenüber anderen Schwenkposition ist diese Verhinderung nicht gegeben. Durch das Schaltsegment, welches gegenüber dem Hauptsegment der Sicherungsvorrichtung verlagerbar, insbesondere verschiebbar ist, kann die Schwenkbeweglichkeit des Sicherungsauslegers verhindert werden, so dass dieser in jener Position fixiert ist, in der er die Beweglichkeit bzw. Zugänglichkeit der Betätigungshandhabe verhindert oder die Abnehmbarkeit der Sicherungsvorrichtung verhindert. Es bedarf dann zunächst einer Verlagerung des Schaltsegments, um nachfolgend den Sicherungsausleger verschwenken zu können und so die Benutzbarkeit des Spenders herzustellen.

Das Schaltsegment ist vorzugsweise translativ gegenüber dem Basisabschnitt des Hauptsegments verschiebbar, wobei diese Beweglichkeit vorzugsweise in beide Bewegungsrichtungen durch Anschläge am Hauptabschnitt limitiert ist.

Es hat sich gezeigt, dass die Kausalität, dass zunächst das Schaltsegment verlagert bzw. verschoben werden muss, um die Benutzbarkeit des Spenders zu gewährleisten, durch Kinder meist schwer zu durchschauen ist. Das Schaltsegment stellt daher einen wirksamen Weg zur Kindersicherung dar.

Von besonderem Vorteil ist es, wenn der Basisabschnitt des Hauptsegments ringförmig ausgebildet ist, wobei er insbesondere derart geformt sein kann, dass er auf der Betätigungshandhabe in der Sicherungsposition aufliegt. Der mindestens eine Sicherungsausleger des Hauptsegments ist vorzugsweise um eine Tangentialachse schwenkbar am Hauptsegment angelenkt.

Das Hauptsegment und der mindestens eine Sicherungsausleger sind vorzugsweise einstückig ausgebildet und über ein Filmscharnier miteinander verbunden.

Von besonderem Vorteil ist eine Gestaltung, bei der eine Mehrzahl von Sicherungsauslegern vorgesehen ist, die um voneinander abweichende Schwenkachsen gegenüber dem Basisabschnitt schwenkbar sind. Sie mehreren Sicherungsausleger, vorzugsweise zwei oder drei Sicherungsausleger, sind vorzugsweise über den Umfang des Basisabschnitts verteilt.

Dabei ist es von besonderem Vorteil, wenn die Beweglichkeit der mehreren Sicherungsausleger gemeinsam durch das nur ein Schaltsegment limitierbar ist. Dies wird insbesondere dadurch erzielt, dass auch das Schaltsegment eine ringförmige Formgebung aufweist und in seiner Sicherungsposition die Mehrzahl von Sicherungsauslegern umgibt, so dass diese nicht voneinander weg nach außen ausgelenkt werden können.

Sowohl Erfindung kann so ausgebildet sein, dass die Sicherungsvorrichtung keine Kappenfunktion übernehmen, sondern lediglich in der genannten Art die Beweglichkeit oder die Zugänglichkeit der Betätigungshandhabe gegenüber dem Mediumspeicher beschränken. Von besonderem Vorteil ist jedoch jeweils eine Gestaltung, bei der auch die Austragöffnung von der Sicherungsvorrichtung überdeckt ist, so dass ein zusätzlicher Schutz besteht. Die genannten Sicherungsvorrichtungen verhindern vorzugsweise formschlüssig die Bewegung der Betätigungshandhabe. Im Falle der zweiten Variante kann dies beispielsweise dadurch erreicht werden, dass am Hauptsegment sowohl für die Betätigungshandhabe als auch für zum Mediumspeicher ortsfeste Teile des Spenders, beispielsweise einen Grundkörper der Fördereinrichtung, Anschlagflächen vorgesehen sind, die eine definierte Beabstandung der Betätigungshandhabe vom Mediumspeicher formschlüssig gewährleisten, so lange die Sicherungsausleger durch das Schaltsegment in ihrer definierten Sicherungsschwenkstellung gehalten werden.

Hinsichtlich der Erfindung ist vorzugsweise vorgesehen, dass alle beschriebenen Teil, insbesondere alle Teile der Sicherungsvorrichtungen, als Kunststoffteile ausgebildet sind.

Da ein erfindungsgemäßer Spender zum Austrag von pharmazeutischen Flüssigkeiten dient, ist er im Auslieferungszustand vorzugsweise mit einer solchen befüllt. Insbesondere ist die Verwendung eines erfindungsgemäßen Spender für pharmazeutische Flüssigkeiten vorgesehen, hinsichtlich derer die Gefahr für Kinder besonders groß ist. Es kann sich hier beispielsweise um Schmerzmittel handeln. Jedoch eignet sich ein erfindungsgemäßer Spender für alle anderen Arten flüssiger und pulverförmiger verschreibungspflichtiger Medikamente.

### KURZBESCHREIBUNG DER ZEICHNUNGEN

Weitere Aspekte und Vorteile der Erfindung ergeben sich außer aus den Ansprüchen auch aus der nachfolgenden Beschreibung bevorzugter Ausführungsbeispiele. Dabei zeigen:
- Fig. 1a und 1b: einen nicht von der Erfindung umfassten Spender in einer ungeschnittenen und einer teilgeschnittenen Darstellung,
- Fig. 2: die Sicherungsvorrichtung des Spenders der Fig. 1a und 1b im entkoppelten Zustand,
- Fig. 3a und 3b: Varianten zum Spender der Fig. 1a, 1b und 2,
- Fig. 4 und 5: einen erfindungsgemäßen Spender in einer teilgeschnittenen Ansicht eines gesicherten Zustandes des Spenders und einer ungeschnittenen Ansicht des entsicherten Zustandes,
- Fig. 6: eine Varianten zum Spender der Fig. 4 und 5.

### DETAILLIERTE BESCHREIBUNG DER AUSFÜHRUNGSBEISPIELE

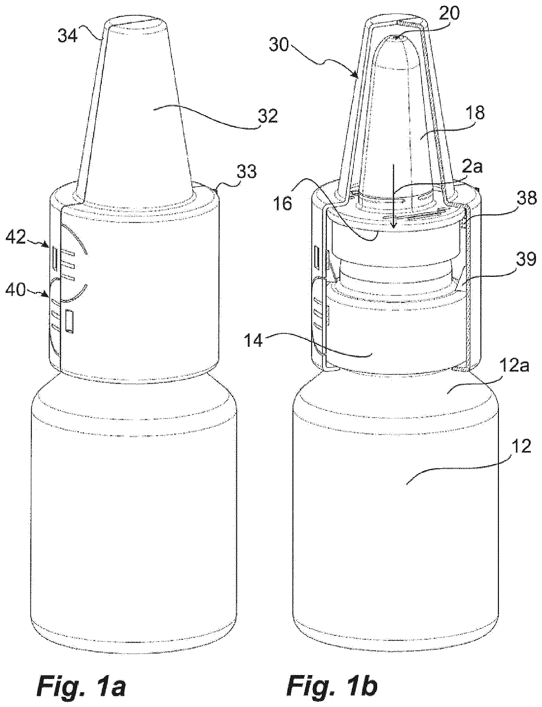

Die Fig. 1a und 1b zeigen einen Spender, der nur zu Erläuterungszwecken hier beschrieben ist, jedoch keine Verwirklichung der Erfindung darstellt. Bezug nehmend auf die teilgeschnittene Darstellung der Fig. 1b findet hier ein Spender Verwendung, der in Hinblick auf seinen Grundaufbau mit bekannten Spendern vergleichbar ist. Dieser Grundaufbau umfasst einen Mediumspeicher 12, eine an einem Hals 12a des Mediumspeichers 12 angebrachte Fördereinrichtung 14 mit einer Betätigungshandhabe 16 zur Betätigung der Fördereinrichtung 14 und eine vorliegend am distalen Ende einer Nasenolive 18 vorgesehene Austragöffnung 20. Durch Verlagerung der Betätigungshandhabe 16 in Richtung des Pfeils 2a kann eine nicht näher dargestellte innerhalb der Fördereinrichtung 14 vorgesehene Kolbenpumpe betätigt werden, die flüssiges Medium aus dem Mediumspeicher 12 zur Austragöffnung 20 fördert.

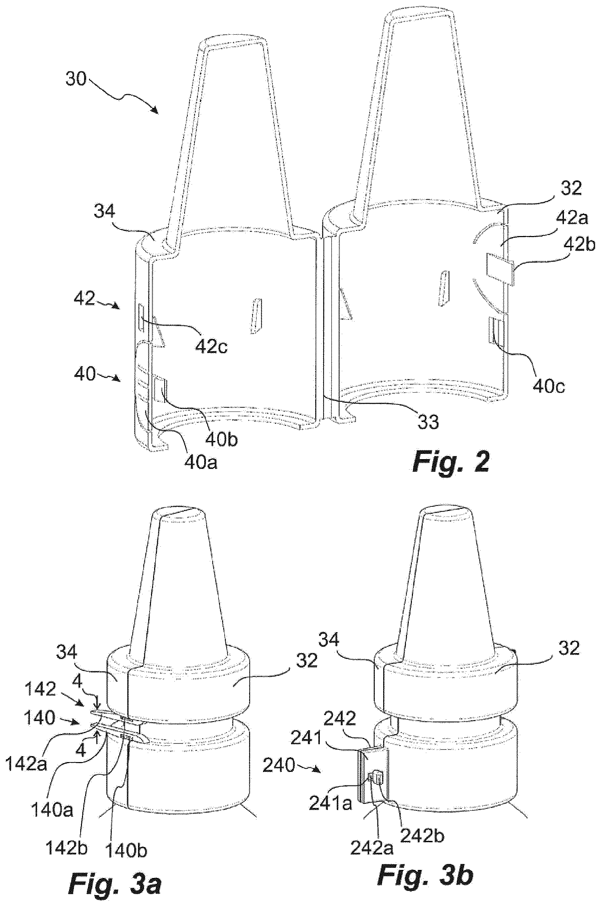

Um die missbräuchliche Verwendung des Spenders durch Kinder zu verhindern, ist eine Sicherungsvorrichtung 30 vorgesehen. Diese Sicherungsvorrichtung 30 verfügt über zwei Teilschalen 32, 34, die durch ein Filmscharnier 33 miteinander verbunden sind. In der durch Fig. 2 verdeutlichten Weise gestattet es dieses Filmscharnier 33, die Teilschalen 32, 34 gegeneinander zu verschwenken, um den geöffneten Zustand der Sicherungsvorrichtung 30 herzustellen. Im geschlossenen Zustand, der in den Fig. 1a und 1b dargestellt ist, sind die Teilschalen 32, 34 gegenüber dem Zustand der Fig. 2 um 180° verschwenkt und durch zwei Kopplungsvorrichtungen 40, 42 im geschlossenen Zustand gesichert.

Die Kopplungsvorrichtungen 40, 42 sind baugleich ausgebildet, unterscheiden sich jedoch hinsichtlich ihrer Verteilung auf die Teilschalen 32, 34. Im Falle der Kopplungsvorrichtung 40 ist ein gegenüber der Teilschale 34 elastisch auslenkbarer Kopplungsabschnitt 40a mit einem Klinkenabschnitt 40b an der Teilschale 34 vorgesehen, während an der anderen Teilschale 32 eine Ausnehmung 40c für den Klinkenabschnitt 40b vorgesehen ist. Im Falle der zweiten Kopplungsvorrichtung 42 ist dagegen der elastisch auslenkbare Abschnitt 42a mit dem Klinkenabschnitt 42b an der Teilschale 32 vorgesehen, während die korrespondierende Ausnehmung 42c an der zweiten Teilschale 34 vorgesehen ist.

Diese Anordnung führt dazu, dass es zum Trennen der Sicherungsvorrichtung 30 von der Fördereinrichtung 14 und der Betätigungshandhabe 16 erforderlich ist, dass beide gegeneinander versetzte Kopplungsabschnitte 40a, 42a gleichzeitig niedergedrückt werden müssen. Erst wenn dies geschehen ist, können die Teilschalen 33, 34 gegeneinander verschwenkt werden, um von der Fördereinrichtung 14 und der Betätigungshandhabe 16 getrennt zu werden und somit die Zugänglichkeit zur Betätigungshandhabe 16 wieder zu ermöglichen. Insbesondere für kleinere Kinder ist es zunächst schwer, zu durchschauen, dass die Sicherungsvorrichtung 30 aufgrund der Tatsache, dass sie die Fördereinrichtung 14 an einem unteren Rand 36 hintergreift, nicht einfach abgezogen werden kann, sondern aufgeschwenkt werden muss. Zudem ist es insbesondere für kleinere Kinder hinsichtlich des Bewegungsablaufes schwer, die beiden Entriegelungsflächen 40a, 42a gleichzeitig zu drücken und dann die erforderliche Aufschwenkbewegung durchzuführen.

Während die Ausgestaltung der Fig. 1a, 1b und 2 vorsieht, dass nicht nur die Fördereinrichtung 14 und die Betätigungshandhabe 16, sondern auch die Nasenolive 18 und damit auch die Austragöffnung 20 von der Sicherungsvorrichtung verdeckt sind, sind auch alternative Gestaltungen denkbar, bei denen die beiden Teilschalen 32, 34 in gekoppeltem Zustand eine gemeinsame Sicherungshülse bilden, die beidseitig offen ist und aus der sich die Nasenolive 18 nach außen erstreckt. Im Falle einer solchen Gestaltung ist es jedoch von Vorteil, wenn an der Innenseite der Teilschalen 32, 34 ein in Fig. 1b gestrichelt dargestellter Fortsatz 38 vorgesehen ist, der dann gemeinsam mit ebenfalls nach innen weisenden Fortsätzen 39, die auf einer Anschlagfläche der Fördereinrichtung 14 aufsitzen, die Verlagerbarkeit der Betätigungshandhabe 16 mechanisch verhindert.

Die Fig. 3a und 3b zeigen alternative Ausgestaltungen der Kopplungsvorrichtung.

Bei der Ausgestaltung der Fig. 3a sind ebenfalls zwei Kopplungsvorrichtungen 140, 142 vorgesehen, wobei diese jeweils einen an der Teilschale 32 angebrachten Schwenkschenkel 140a, 142a mit Ausnehmung 140b, 142b umfassen. An der anderen Teilschale 34 sind korrespondierende Nocken vorgesehen, die im dargestellten gekoppelten Zustand der Teilschalen 32, 34 in den Ausnehmungen 140b, 142b der Schwenkschenkel 140a, 142a angeordnet sind. Zum Entkoppeln müssen die Schwenkschenkel 140a, 142a in Richtung der Pfeile 4 aufeinander zu bewegt werden.

Bei der Gestaltung gemäß Fig. 3b ist an den Teilschalen 32, 34 eine Kopplungsvorrichtung 240 mit Kopplungsflanschen 241, 242 vorgesehen, wobei im Kopplungsflansch 241 eine Ausnehmung 241a vorgesehen ist und wobei am anderen Kopplungsflansch 242 ein Kopplungsfortsatz 242a mit einem aufgeweiteten Endbereich 242b vorgesehen ist. Im gekoppelten Zustand der Fig. 3b ragt der Kopplungsfortsatz 242a durch die Kopplungsdurchbrechung 241a hindurch. Da der aufgeweitete Endbereich 242b im untordierten Zustand des Fortsatzes 242a breiter ist als die Breite der Durchbrechung 241a, ist somit ein zuverlässig gekoppelter Zustand erzielbar. Zum Entkoppeln muss der Endbereich 242b Kopplungsfortsatz 242a um etwa 90° tordiert werden, bis der aufgeweitete Endbereich 242b durch die Durchbrechung 241a hindurchpasst. Eine solche Torsionsbewegung ist für Kinder sowohl hinsichtlich des Verständnisses als auch hinsichtlich der motorischen Fähigkeiten schwer zu realisieren.

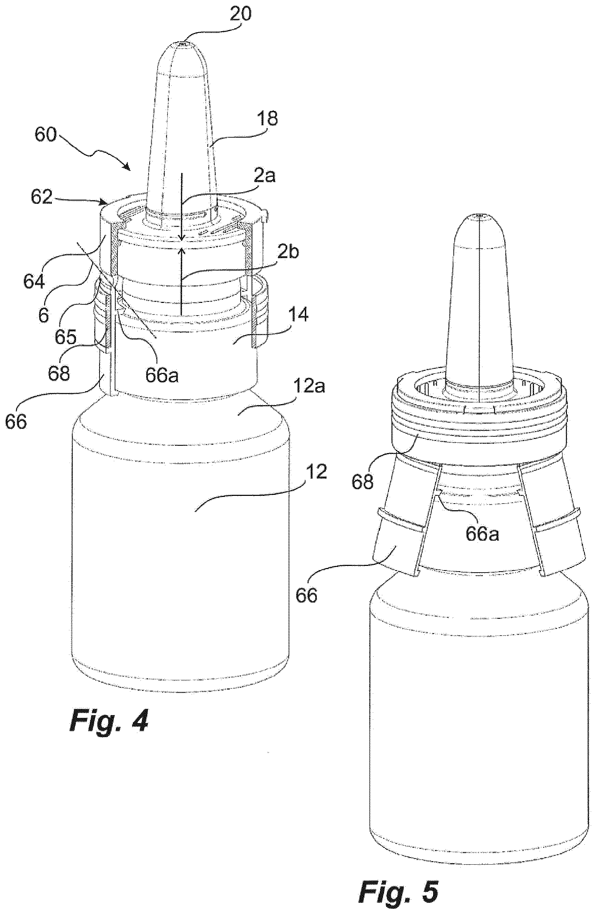

Die Fig. 4 bis 5 zeigen einen Spender gemäß der Erfindung. Der Grundaufbau dieses Spenders mit Mediumspeicher 12, Fördereinrichtung 14, Betätigungshandhabe 16 sowie Nasenolive 18 mit Austragöffnung 20 an deren distalen Ende ist gegenüber dem Spender der vorangegangenen Figuren vergleichbar. Die Sicherungsvorrichtung 60 gemäß dem Spender der Fig. 4 und 5 ist jedoch deutlich anders geartet. Diese Sicherungsvorrichtung 60 weist zwei separate Bauteile auf, nämlich ein Hauptsegment 62 sowie ein Schaltsegment 68. Das Hauptsegment 62 verfügt über einen Basisabschnitt 64, der in der Fig. 4 gut zu entnehmenden Weise fest mit der Betätigungshandhabe 16 verbunden ist. Der Basisabschnitt 64 weist eine ringartige Gestalt auf. Am unteren Ende des Basisabschnitts 64 finden sich insgesamt drei schwenkbare Sicherungsausleger 66, welche jeweils mit Filmscharnieren 65 am Basisabschnitt 64 um tangential ausgerichtete Schwenkachsen 6 verschwenkbarsind.

Während Fig. 4 eine Sicherungsschwenkposition der Sicherungsausleger 66 zeigt, zeigt Fig. 5 eine entsicherte Schwenkstellung. In Fig. 4 ist gut zu erkennen, dass an der Innenseite der Sicherungsausleger 66 Stützflächen 66a vorgesehen sind, die im gesicherten Zustand der Fig. 4 derart mit dem Grundkörper der Fördereinrichtung 14 zusammenwirken, dass eine Verlagerung der Betätigungshandhabe 16, die nur gemeinsam mit dem Basisabschnitt 64 verlagert werden kann, in Richtung des Pfeils 2a nicht möglich ist. Eine solche Bewegung ist erst dann möglich, wenn die Sicherungsausleger 66 ihre in Fig. 5 dargestellte Stellung einnehmen. Im gesicherten Zustand der Fig. 4 wird dieses Ausschwenken durch das Schaltsegment 68 verhindert. Das Schaltsegment 68 ist als Ring ausgestaltet, der außenseitig am Hauptsegment 62 vorgesehen ist und zwischen einer oberen Endlage der Fig. 5 und einer unteren Endlage der Fig. 4 verschiebbar ist. In der unteren Endlage der Fig. 4 sichert das Schaltsegment 68 die Sicherungsausleger 66 und verhindert deren Auslenkung nach außen. Es bedarf zunächst einer Verlagerung des Schaltsegments 68 in Richtung des Pfeils 2b nach oben, bis dieses die Position der Fig. 5 einnimmt. Erst dann können die Sicherungsausleger 66 nach außen verschwenkt werden und gestatten nachfolgend unbehindert eine Betätigung durch Verlagerung der Betätigungshandhabe und der gesamten Sicherungsvorrichtung 60 in Richtung des Pfeils 2a.

Der komplexe Bewegungsablauf ist zumindest für kleinere Kinder kaum zu bewerkstelligen, insbesondere da die Bewegungsrichtung des Schaltsegments 68 zum Entsichern der Sicherungsvorrichtung 60 in gegenüber der eigentlichen Betätigungsrichtung 2a entgegengesetzter Richtung 2b zu erfolgen hat.

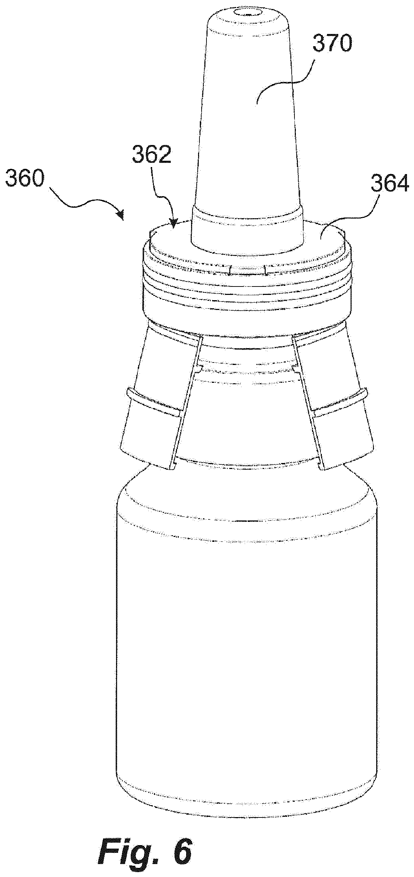

Fig. 6 zeigt eine Abwandlung der Ausgestaltung der Fig. 4 und 5, die sich dadurch unterscheidet, dass hier der Basisabschnitt 364 des Hauptsegments 362 der Sicherungsvorrichtung 360 nicht fest mit der Betätigungshandhabe 16 verbunden ist. Stattdessen umfasst die Sicherungsvorrichtung im Falle der Ausgestaltung der Fig. 6 auch einen Kappenabschnitt 370, der die Austragöffnung 20 überdeckt. Bei dieser Gestaltung ist vorgesehen, dass nach Entsicherung in Art, wie zu den Fig. 4 und 5 beschrieben, die Sicherungsvorrichtung 360 als Ganzes und einschließlich des Kappenabschnitts 370 abgenommen wird, um dann einen Austragvorgang zu gestatten.

## Patentansprüche

1. Spender zum Austrag eines flüssigen oder pulverförmigen Mediums mit
- einem Mediumspeicher (12),
- einer Austragöffnung (20) und
- einer Fördereinrichtung (14) zur Förderung von Medium vom Mediumspeicher (12) zur Austragöffnung (20),
wobei
- die Fördereinrichtung (14) über eine gegenüber dem Mediumspeicher (12) in einer Betätigungsrichtung (2a) beweglichen Betätigungshandhabe (16) verfügt, mittels derer die Fördereinrichtung (14) zum Zwecke des Austrags betätigt werden kann, und
- eine Sicherungsvorrichtung (60) vorgesehen ist, die über ein Hauptsegment (62) und ein Schaltsegment (68) verfügt, und
- die Sicherungsvorrichtung (60) derart an der Fördereinrichtung (14) anbringbar ist oder angebracht ist, dass bei Sicherung des Hauptsegments (62) mittels des Schaltsegments (68) die Zugänglichkeit und/oder die Beweglichkeit der Betätigungshandhabe (16) gegenüber dem Mediumspeicher (12) verhindert wird und/oder die Sicherungsvorrichtung (60) nicht von der Fördereinrichtung (14) trennbar ist und
**dadurch gekennzeichnet, dass**
- das Hauptsegment (62) einen Basisabschnitt (64) und eine Mehrzahl von Sicherungsauslegern (66) aufweist, die um voneinander abweichende Schwenkachsen (6) gegenüber dem Basisabschnitt (64) schwenkbar sind,
- das Schaltsegment (68) gegenüber dem Hauptsegment (62) zwischen einer Sicherungsposition und einer Freigabeposition verlagerbar ist, wobei das Schaltsegment (68) die Beweglichkeit der Sicherungsausleger (66) des Hauptsegments (62) in der Sicherungsposition limitiert und in der Freigabeposition nicht limitiert, und
- die Sicherungsvorrichtung (60) derart ausgebildet ist, dass durch Verlagerung des Schaltsegments (68) in die Freigabeposition und die dadurch erzielbare Beweglichkeit der Sicherungsausleger (66) die Zugänglichkeit oder Beweglichkeit der Betätigungshandhabe (16) hergestellt wird und/oder die Sicherungsvorrichtung (60) von der Fördereinrichtung (14) trennbar ist.

2. Spender nach Anspruch 1,
**dadurch gekennzeichnet, dass**
der Basisabschnitt (64) des Hauptsegments (62) ringförmig ausgebildet ist und die Sicherungsausleger (66) des Hauptsegments (62) jeweils um eine Tangentialachse (6) schwenkbar am Hauptsegment (62) angelenkt sind.

3. Spender nach Anspruch 1 oder 2,
**dadurch gekennzeichnet, dass**
die Sicherungsvorrichtung (60; 360) einen Kappenabschnitt (370) aufweist, der im Sicherungszustand die Austragöffnung (20) überdeckt.

4. Spender nach einem der vorstehenden Ansprüche,
**dadurch gekennzeichnet, dass**
die Sicherungsvorrichtung (30; 60) im Sicherungszustand formschlüssig eine Verlagerung der Betätigungshandhabe (16) gegenüber dem Mediumspeicher (12) verhindert.

## Claims

1. Dispenser for discharging a liquid or pulverulent medium, having
- a medium reservoir (12);
- a discharge opening (20); and
- a conveying installation (14) for conveying medium from the medium reservoir (12) to the discharge opening (20),
wherein
- the conveying installation (14) possesses an activation handle (16) which is movable in an activation direction (2a) in relation to the medium reservoir (12) and by means of which the conveying installation (14) can be activated for the purpose of discharging, and
- a safeguard device (60) which possesses a main segment (62) and a switching segment (68) is provided; and
- the safeguard device (60) is capable of being attached or is attached to the conveying installation (14) in such a manner that, when securing the main segment (62) by means of the switching segment (68), the accessibility and/or the mobility of the activation handle (16) in relation to the medium reservoir (12) is impeded and/or the safeguard device (60) is incapable of being separated from the conveying installation (14); and
**characterized in that**
- the main segment (62) has a base portion (64) and a plurality of securing outriggers (66) which in relation to the base portion (64) are pivotable about mutually deviating pivot axes (6);
- the switching segment (68) in relation to the main segment (62) is repositionable between a securing position and a releasing position, wherein the switching segment (68) in the securing position limits the mobility of the securing outriggers (66) of the main segment (62) and in the releasing position does not limit said mobility; and
- the safeguard device (60) is configured in such a manner that the accessibility or mobility of the activation handle (16) is established by repositioning the switching element (68) to the releasing position and by the mobility achievable on account thereof, and/or the safeguard device (60) is capable of being separated from the conveying installation (14).

2. Dispenser according to Claim 1,
**characterized in that**
the base portion (64) of the main segment (62) is configured so as to be annular, and the securing outriggers (66) of the main segment (62) are in each case articulated on the main segment (62) so as to be pivotable about a tangential axis (6).

3. Dispenser according to Claim 1 or 2,
**characterized in that**
the safeguard device (60; 360) has a cap portion (370) which in the securing state covers the discharge opening (20).

4. Dispenser according to one of the preceding claims,
**characterized in that**
the safeguard device (30; 60) in the securing state in a form-fitting manner impedes any repositioning of the activation handle (16) in relation to the medium reservoir (12).

## Revendications

1. Distributeur pour délivrer un milieu liquide ou pulvérulent, comprenant :
- un dispositif de stockage de milieu (12),
- une ouverture de décharge (20) et
- un dispositif de transport (14) pour transporter le milieu depuis le dispositif de stockage de milieu (12) jusqu'à l'ouverture de décharge (20),
dans lequel distributeur :
- le dispositif de transport (14) dispose d'une manette d'actionnement (16) déplaçable dans une direction d'actionnement (2a) par rapport au dispositif de stockage de milieu (12), au moyen de laquelle le dispositif de transport (14) peut être actionné en vue de la décharge et
- un dispositif de fixation (60) est prévu, lequel dispose d'un segment principal (62) et d'un segment de commutation (68), et
- le dispositif de fixation (60) peut être monté ou est monté sur le dispositif de transport (14) de telle sorte que lors de la fixation du segment principal (62) au moyen du segment de commutation (68), l'accès et/ou le déplacement de la manette d'actionnement (16) par rapport au dispositif de stockage de milieu (12) soient empêchés et/ou de telle sorte que le dispositif de fixation (60) ne puisse pas être séparé du dispositif de transport (14) et
**caractérisé en ce que**
- le segment principal (62) présente une portion de base (64) et une pluralité de bras de fixation (66) qui peuvent pivoter autour d'axes de pivotement (6) différents les uns des autres par rapport à la portion de base (64),
- le segment de commutation (68) peut être déplacé par rapport au segment principal (62) entre une position de fixation et une position de libération, le segment de commutation (68) limitant la mobilité des bras de fixation (66) du segment principal (62) dans la position de fixation et ne la limitant pas dans la position de libération, et
- le dispositif de fixation (60) étant réalisé de telle sorte que du fait du déplacement de l'élément de commutation (68) dans la position de libération et du fait de la mobilité des bras de fixation (66) pouvant ainsi être obtenue, l'accès à la manette d'actionnement (16) ou sa capacité de mouvement sont permis et/ou le dispositif de fixation (60) peut être séparé du dispositif de transport (14).

2. Distributeur selon la revendication 1,
**caractérisé en ce que**
la portion de base (64) du segment principal (62) est réalisée sous forme annulaire et les bras de fixation (66) du segment principal (62) sont articulés au segment principal (62) de manière à pouvoir chacun pivoter autour d'un axe tangentiel (6) .

3. Distributeur selon la revendication 1 ou 2,
**caractérisé en ce que**
le dispositif de fixation (60 ; 360) présente une portion de capuchon (370) qui recouvre l'ouverture de décharge (20) dans l'état de fixation.

4. Distributeur selon l'une quelconque des revendications précédentes,
**caractérisé en ce que**
le dispositif de fixation (30 ; 60), dans l'état de fixation, empêche, par engagement par correspondance de formes, un déplacement de la manette d'actionnement (16) par rapport au dispositif de stockage de milieu (12).
